# EUROPÄISCHE PATENTANMELDUNG

(11) **EP 3 799 890 A1**
(43) Veröffentlichungstag der Anmeldung: **07.04.2021**
(21) Anmeldenummer: 20199502.4
(22) Anmeldetag: 01.10.2020
(51) Int. Cl.: A61L 2/24, A47L 15/00, A61L 2/07, A61L 2/18

(54) **AUTOMATISCHE STERILISATIONSVORRICHTUNG-KAMMERREINIGUNG**

(30) Priorität: 01.10.2019 DE 102019126428
(71) Anmelder: MMM Münchener Medizin Mechanik GmbH, 82152 Planegg (DE)
(72) Erfinder: Eibl, Robert, 92539 Schönsee (DE); Vogt, Alexander, 86163 Augsburg (DE); Bönisch, Martin, 80687 München (DE); Göllner, Gerald, 92539 Schönsee (DE)
(74) Vertreter: Wittmann, Ernst-Ulrich

(57) **Zusammenfassung**

Die Erfindung betrifft eine Sterilisationsvorrichtung (100), insbesondere einen Dampfsterilisator. Die Sterilisationsvorrichtung (100) umfasst eine Sterilisationskammer (110) zur Aufnahme von zu sterilisierendem Gut in einer Normalbetriebsart, wenigstens eine Einspritzvorrichtung zum Einspritzen eines Reinigungsmittels in die Sterilisationskammer (110), wenigstens eine Ablaufvorrichtung zum Abführen des Reinigungsmittels aus der Sterilisationskammer (110), und eine Steuervorrichtung, die dazu eingerichtet ist, während einer Reinigungsbetriebsart Reinigungsmittel zum Reinigen der Sterilisationskammer (110) mittels der Einspritzvorrichtung in die Sterilisationskammer (110) einzuspritzen.

Die Erfindung betrifft des Weiteren ein Verfahren zum Reinigen einer Sterilisationskammer (110) eines Sterilisationsgeräts.

## Beschreibung

Die vorliegende Anmeldung betrifft eine Sterilisationsvorrichtung, insbesondere einen Dampfsterilisator, umfassend eine Sterilisationskammer zur Aufnahme von zu sterilisierendem Gut, sowie ein Verfahren zum Reinigen einer Sterilisationskammer eines Sterilisationsgeräts.

Moderne Dampfsterilisatoren, auch bekannt als Autoklaven, sind Anlagen, die in verschiedenen Industriezweigen, wie zum Beispiel im Gesundheitswesen, der Pharmaindustrie, Versuchslaboren usw. eingesetzt werden. Wesentliche medienberührende Komponenten von derartigen und anderen Sterilisationsvorrichtungen sind Dampfleitungen sowie die Sterilisationskammer, die das zu sterilisierende Gut aufnimmt. Derartige Anlagenteile werden häufig aus hochlegierten, korrosionsbeständigen Werkstoffen wie insbesondere Chrome/Nickel/Molybdän-Stählen gefertigt, beispielsweise solche mit den Werkstoffnummern 1.4404 oder 1.4435 gemäß DIN EN 10088-1:2014-12.

Obwohl derartige Werkstoffe als korrosionsarm bzw. rostfrei bezeichnet werden, kommt es im praktischen Einsatz, insbesondere unter Betriebsbedingungen wie sie in Sterilisationsvorrichtungen auftreten, zu ungewünschten Oberflächenveränderungen. Die Oberflächenveränderungen bestehen vor allem aus Eisenoxiden, die typischerweise einen roten bzw. rötlichbraunen bis schwarzen Belag bilden. Dieser Effekt wird teilweise auch mit dem englischen Begriff "Rouging" bezeichnet.

Die genannten Oberflächenveränderungen sind in mehrfacher Hinsicht problematisch. Zum einen reduzieren sie eine Schutzschicht des eigentlich korrosionsbeständigen Werkstoffes und beschleunigen somit eine weitere Korrosion der betroffenen Bauteile. Zum anderen verändern sie deren Oberflächenbeschaffenheit, sodass sich möglicherweise weitere Verunreinigungen im Bereich der Oberfläche festsetzen können, die eine Funktion der Sterilisationsvorrichtung beeinträchtigen könnten. Auch wenn die Eisenoxide selbst als hygienisch unbedenklich gelten, verhindern sie zumindest eine optische Inspektion der Sauberkeit der Sterilisationskammer und werden daher von Nutzern als visuell störend empfunden.

Unter anderem aus diesen Gründen geben Hersteller von Sterilisationsgeräten im Rahmen ihrer Gerätedokumentation in der Regel Empfehlungen dazu ab, wie und in welchen Intervallen die von Ihnen vertriebenen Geräte gereinigt und gepflegt werden sollen. Grundsätzlich möglich ist beispielsweise der Einsatz von sogenannten Edelstahlreinigern oder ein regelmäßiges Auswischen der Sterilisationskammer mit vollentsalztem Wasser. Derartige Verfahren sind beispielsweise in dem Informationsblatt mit dem Titel "Information zu Edelstahloberflächen in Dampfsterilisatoren" des "Arbeitskreis Kammer" aus dem Jahr 2017 beschrieben.

Bei stärker ausgeprägten Oberflächenbelegen ist eine derartige, manuelle Reinigung in der Regel jedoch nicht ausreichend, um eine vollständige Entfernung der Belege zu ermöglichen. Um solche Belege dennoch weitgehend entfernen zu können, sind aus dem Stand der Technik weitere Verfahren zur Behandlung von Chrom/Nickel/Molybdän-Stählen bekannt. Wegen der verwendeten Chemikalien und durchgeführten Verfahrensschritten benötigen diese Verfahren in der Regel spezielle Reinigungsausrüstung und einen geschulten Bediener für die Durchführung der Reinigung. Die damit verbundene Rüstzeiten und der zur Reinigung erforderliche Aufwand bedeuten in der Praxis, dass ein langfristiger Ausfall der zur reinigenden Sterilisationskammer unumgänglich ist.

Es ist eine Aufgabe der vorliegenden Erfindung, alternative Vorrichtungen und Verfahren zum Reinigen von Sterilisationsvorrichtungen mit einer Sterilisationskammer anzugeben. Bevorzugt sollen die beschriebenen Verfahren und Vorrichtungen eine zeit- und ressourcensparende Reinigung ermöglichen. Ebenfalls bevorzugt ist, wenn zu deren Durchführung kein besonders geschultes Personal oder längerer Ausfallzeiten der Sterilisationsvorrichtung erforderlich sind.

Die vorliegende Erfindung löst oder mindert die oben genannten Probleme durch die Vorsehung einer in die Sterilisationsvorrichtung weitgehend integrierten Reinigungsvorrichtung und eines vollautomatisch durchführbaren Reinigungsprozesses. Durch die Integration und Automatisierung kann der beschriebene Reinigungsprozess weitgehend unbeaufsichtigt und in regelmäßigen Abständen, beispielsweise in relativ kurzen Betriebspausen der Sterilisationsvorrichtung durchgeführt werden.

Hierzu ist gemäß einer ersten Ausgestaltung eine Sterilisationsvorrichtung, insbesondere ein Dampfsterilisator vorgesehen, der eine Sterilisationskammer zur Aufnahme von zu sterilisierendem Gut in einer Normalbetriebsart, wenigstens eine Einspritzvorrichtung zum Einspritzen eines Reinigungsmittels in die Sterilisationskammer, wenigstens eine Ablaufvorrichtung zum Abführen des Reinigungsmittels aus der Sterilisationskammer und eine Steuervorrichtung umfasst, die dazu eingerichtet ist, während einer Reinigungsbetriebsart Reinigungsmittel zum Reinigen der Sterilisationskammer mittels der Einspritzvorrichtung in die Sterilisationskammer einzuspritzen.

Durch Vorsehung wenigstens einer Einspritzvorrichtung und wenigstens einer Ablaufvorrichtung im Bereich der Sterilisationskammer kann auf ein manuelles Auftragen und Entfernen von Reinigungsmitteln verzichtet werden. Dies erfolgt stattdessen vollautomatisch in einer Reinigungsbetriebsart durch eine Steuervorrichtung, wie beispielsweise eine ohnehin vorhandene Prozesssteuerung der Sterilisationsvorrichtung. Durch die Automatisierung kann insbesondere ein zeitlicher Abstand zwischen durchgeführten Reinigungen reduziert werden. Dies ergibt den zusätzlichen Vorteil, dass das eingesetzte Reinigungsmittel chemisch weniger aggressiv sein kann als die bei bekannten Reinigungsverfahren für Edelstähle eingesetzten Reinigungsmittel. Auch auf eine direkte mechanische Einwirkung auf die Oberfläche kann in der Regel verzichtet werden. Zudem erlaubt der verhältnismäßig einfache Aufbau der zur Reinigung erforderlichen Komponenten eine vollständige Integration der Reinigungsvorrichtung in die Sterilisationsvorrichtung.

In wenigstens einer Ausgestaltung umfasst die Einspritzvorrichtung wenigstens eine Pumpe und wenigstens eine Düse zum Einspritzen eines flüssigen Reinigungsmittels in die Sterilisationskammer. Eine derartige Anordnung ist mechanisch relativ einfach aufgebaut und ermöglicht ein einfaches, gleichmäßiges Benetzen einer Edelstahloberfläche einer Sterilisationskammer mit dem Reinigungsmittel.

In verschiedenen Ausgestaltungen ist die wenigstens eine Düse innerhalb der Sterilisationskammer in einem Bereich außerhalb eines Nutzraums der Sterilisationskammer fest montiert und/oder versenkbar in eine Wand der Sterilisationskammer eingebaut. Auf diese Weise wird der zur Verfügung stehende Nutzraum der Sterilisationsvorrichtung nicht reduziert. Insbesondere kann eine Düse in ohnehin zur Verfügung stehenden Seitenräume eingebaut werden, in denen typischerweise kein zu sterilisierendes Gut aufgenommen wird.

In wenigstens einer Ausgestaltung ist die wenigstens eine Düse an einer Seitenwand der Sterilisationskammer angeordnet. Dies hat neben der oben beschriebenen Platzersparnis den zusätzlichen Vorteil, dass gegebenenfalls im direkten Umfeld der Düse vorhandene Abschattungsbereiche, die nicht direkt von der Düse angespritzt werden, durch Einwirkung der Schwerkraft mittels des von oben herablaufenden Reinigungsmittels benetzt werden.

In verschiedenen Ausgestaltungen rotiert wenigstens eine Düse beim Einspritzen des Reinigungsmittels und/oder ist die wenigstens eine Düse auf einem rotierenden Sprüharm angeordnet. Durch die Rotation der Düse kann eine besonders gleichmäßige und vollständige Benetzung der Oberfläche der Sterilisationskammer bewirkt werden. Dabei ist es sowohl möglich, eine Düse mit einem festen Auslass auf einem rotierenden Sprüharm anzuordnen oder eine ortsfest montierte Düse mit einem rotierenden Düsenkopf zu verwenden.

In wenigstens einer Ausgestaltung weist die wenigstens eine Düse einen Sprühwinkel von mehr als 180°, bevorzugt mehr als 270° auf, um einen möglichst großen Teil der Sterilisationskammer direkt mit dem Reinigungsmittel zu benetzen.

In wenigstens einer Ausgestaltung umfasst die Einspritzvorrichtung eine Mehrzahl von räumlich versetzt und/oder verdreht angeordneten Düsen. Dies ermöglicht insbesondere, Abschattungsbereiche einer Düse durch eine andere Düse abzudecken und/oder die Abstände zwischen den einzelnen Düsen und zu benetzenden Oberflächen zu reduzieren.

In wenigstens einer Ausgestaltung bilden die wenigstens eine Ablaufvorrichtung, die wenigstens eine Pumpe und die wenigstens eine Düse zusammen einen Umwälzkreislauf zum wiederholten Benetzen der Innenseite der Sterilisationskammer mit dem flüssigen Reinigungsmittel. Das wiederholte Auftragen des Reinigungsmittels in einem Umwälzkreislauf reduziert den Verbrauch von Reinigungsmitteln und ermöglicht eine wiederholte, besonders sanfte Reinigung der Innenseite der Sterilisationskammer.

In wenigstens einer Ausgestaltung sind die wenigstens eine Ablaufvorrichtung und die wenigstens eine Pumpe über wenigstens ein erstes Ventil miteinander verbunden, wobei das wenigstens eine erste Ventil in der Reinigungsbetriebsart eine Rückführung des Reinigungsmittels über den Umwälzkreislauf in die Sterilisationskammer ermöglicht und in der Normalbetriebsart eine Rückführung von ablaufenden Flüssigkeiten in die Sterilisationskammer unterbindet. Durch ein derartiges Ventil kann der Umwälzkreislauf in einer Normalbetriebsart der Sterilisationsvorrichtung von der Sterilisationskammer getrennt werden, um die Ausbreitung von Keimen oder sonstigen Verschmutzungen innerhalb der Sterilisationskammer zu unterbinden.

In wenigstens einer Ausgestaltung umfasst die Sterilisationsvorrichtung weiter eine Dosiervorrichtung, wobei die Steuervorrichtung dazu eingerichtet ist, die Dosiervorrichtung derart anzusteuern, dass in der Reinigungsbetriebsart eine vorbestimmte Menge des Reinigungsmittels in die Sterilisationskammer eingeleitet wird.

In wenigstens einer Ausgestaltung umfasst die Dosiervorrichtung wenigstens einen Vorratsbehälter zur Aufnahme wenigstens einer Chemikalie und wenigstens eine mit dem Vorratsbehälter verbundene Dosiereinrichtung, insbesondere eine Dosierpumpe und/oder ein zweites Ventil. Eine derartige Dosiervorrichtung erlaubte eine vollautomatische Anmischung und/oder Einleitung einer vorbestimmten Menge des Reinigungsmittels.

In wenigstens einer Ausgestaltung ist der Vorratsbehälter zur Aufnahme eines Reinigerkonzentrats und/oder eines ersten Reaktants zur Herstellung des Reinigungsmittels eingerichtet, und die Dosiervorrichtung umfasst des Weiteren eine Mischvorrichtung zum Mischen des Reinigerkonzentrats mit einem Verdünnungsmittel, insbesondere Wasser, bzw. des ersten Reaktants mit wenigstens einem zweiten Reaktant zur Herstellung des Reinigungsmittels in einem vorbestimmten Verhältnis. Ein Anmischen, insbesondere Verdünnen eines Reinigerkonzentrats, erlaubt eine platzsparende Bereitstellung von größeren Mengen des Reinigungsmittels und somit einen unbeaufsichtigten Betrieb der Sterilisationsvorrichtung über einen längeren Zeitraum.

In wenigstens einer Ausgestaltung ist die Dosiervorrichtung direkt mit der Einspritzvorrichtung verbunden und die vorbestimmte Menge des Reinigungsmittels wird von der Dosiervorrichtung über die Einspritzvorrichtung in die Sterilisationskammer eingebracht. In dieser Ausgestaltung ist neben dem Auslass der Einspritzvorrichtung und der Ablaufvorrichtung keine weitere Öffnung in der Sterilisationskammer erforderlich, was insbesondere eine Reinigung der Sterilisationskammer erleichtert.

In wenigstens einer alternativen Ausgestaltung ist die Dosiervorrichtung über einen von der Einspritzvorrichtung gesonderten Zulauf mit der Sterilisationskammer verbunden und die vorbestimmte Menge des Reinigungsmittels wird von der Dosiervorrichtung über den Zulauf in die Sterilisationskammer eingebracht. Ein derartiger Aufbau ermöglicht eine einfache mechanische Konstruktion der Sterilisationsvorrichtung, in der insbesondere mit Druck beaufschlagte Teile eines Umwälzkreislaufes hydraulisch von der Dosiervorrichtung getrennt sind.

In wenigstens einer Ausgestaltung umfasst die Sterilisationsvorrichtung wenigstens einen Sensor, wobei die Steuervorrichtung des Weiteren dazu eingerichtet ist, den Druck bzw. die Temperatur des Reinigungsmittels in Abhängigkeit von erfassten Sensordaten während der Reinigungsbetriebsart zu regeln. Eine derartige Druckbeziehungsweise Temperaturregelung ermöglicht die Optimierung des Reinigungsprozesses für einen bestimmten Druck bzw. eine bestimmte Temperatur des Reinigungsmittels.

In wenigstens einer Ausgestaltung ist die Sterilisationskammer beheizbar, insbesondere durch Einleiten von Heißdampf in einen Doppelmantel der Sterilisationskammer und/oder durch direktes Einleiten von Heißdampf in die Sterilisationskammer. Die Steuervorrichtung regelt die Temperatur des Reinigungsmittels zumindest teilweise über eine Beheizung der Sterilisationskammer. Ein derartiger Aufbau ermöglicht eine einfache Temperaturregelung bzw. Beheizung des Reinigungsmittels durch ohnehin in üblichen Sterilisationsvorrichtungen verbaute Komponenten.

In wenigstens einer Ausgestaltung umfasst die Einspritzvorrichtung ein von der Sterilisationskammer gesondertes Heizelement zum Beheizen des Reinigungsmittels vor dem Einspritzen in die Sterilisationskammer, und die Steuervorrichtung regelt die Temperatur des Reinigungsmittels zumindest teilweise über eine Beheizung des Reinigungsmittels durch das Heizelement. Eine derartige, direkt auf das Reinigungsmittel einwirkende Beheizung ermöglicht eine besonders präzise Temperaturregelung und vermeidet eine unnötige Beheizung der gesamten Sterilisationskammer.

In wenigstens einer Ausgestaltung umfasst die Einspritzvorrichtung des Weiteren ein Regelventil zur Druckregulierung, und die Steuervorrichtung ist dazu eingerichtet, den Druck des eingespritzten Reinigungsmittels über das Regelventil zu regeln. Auf diese Weise kann insbesondere der Druck, mit dem das eingesetzte Reinigungsmittel auf die Oberfläche der Sterilisationskammer trifft, genau gesteuert werden, was eine weitere Optimierung des Reinigungsprozesses ermöglicht.

In wenigstens einer Ausgestaltung umfasst die Sterilisationsvorrichtung wenigstens eine Spülvorrichtung zum Spülen der Sterilisationskammer mit einem chemisch neutralen Spülmittel, insbesondere Wasser, wobei die Steuervorrichtung des Weiteren dazu eingerichtet ist, die Sterilisationskammer nach dem Einspritzen des Reinigungsmittels mit dem Spülmittel zu spülen. Eine derartige Spülvorrichtung ermöglicht es, die Sterilisationsvorrichtung nach Durchführung der Reinigung wieder für die Sterilisation von zu sterilisierendem Gut vorzubereiten. Insbesondere können sämtliche zur Reinigung eingesetzten Chemikalien aus dem Innenraum der Sterilisationskammer entfernt werden.

In wenigstens einer Ausgestaltung ist die Steuervorrichtung als programmierbare Prozesssteuerung ausgestaltet, die zur Durchführung wenigstens eines Reinigungsprogramms und wenigstens eines Sterilisationsprogramms programmiert ist. Eine derartige, programmierbare Prozesssteuerung ist in den meisten Sterilisationsvorrichtungen ohnehin zur Durchführung von Sterilisationsprogrammen vorgesehen. Die weitere Programmierung der Prozesssteuerung auch zur Durchführung wenigstens eines Reinigungsprogramms reduziert den zusätzlichen Bauteileaufwand und ermöglicht somit eine kostengünstige, gegebenenfalls auch nachträgliche, Integration der Reinigungsvorrichtung in die Sterilisationsvorrichtung.

In wenigstens einer Ausgestaltung ist die Prozesssteuerung dazu eingerichtet, das wenigstens eine Reinigungsprogramm regelmäßig, insbesondere nach einer vorbestimmten Anzahl von durchgeführten Sterilisationsprogrammen, nach Ablauf einer vorbestimmten Zeitspanne und/oder zu einem vorbestimmten, wiederkehrenden Zeitpunkt durchzuführen. Eine regelmäßige, insbesondere benutzungs- oder tageszeitabhängige, Reinigung der Sterilisationsvorrichtung verhindert den Aufbau dicker Oberflächenbeläge und sorgt dafür, dass die Sterilisationskammer stets in einem visuell einwandfreien Zustand gehalten wird.

Die oben genannte Aufgabe wird des Weiteren durch ein Verfahren zum Reinigen einer Sterilisationskammer eines Sterilisationsgeräts, insbesondere zum Entfernen von Eisenoxiden von einer Edelstahloberfläche der Sterilisationskammer, mit den folgenden Schritten gelöst:
- Benetzen einer Innenseite der Sterilisationskammer mit einem über eine in die Sterilisationskammer integrierte Einspritzvorrichtung eingespritzten Reinigungsmittel; und
- Abführen des in die Sterilisationskammer eingespritzten Reinigungsmittels über wenigstens eine Ablaufvorrichtung der Sterilisationskammer, wobei die Schritte des Benetzens und Abführens durch eine Prozesssteuerung des Sterilisationsgeräts wiederholt durchgeführt werden.

Das wiederholte, durch eine Prozesssteuerung durchgeführte Benetzen der Innenseite der Sterilisationskammer und Abführen des dabei verwendeten Reinigungsmittels ermöglicht eine sanfte, wiederkehrende und somit besonders zuverlässige Reinigung einer Sterilisationskammer.

In wenigstens einer Ausgestaltung umfasst das eingespritzte Reinigungsmittel eine Säure, insbesondere eine wässrige Lösung, insbesondere mit einem pH-Wert von weniger als fünf, bevorzugt mit einem pH-Wert in einem Bereich zwischen zwei und vier. Ein derartiges, saures Reinigungsmittel eignet sich insbesondere zum Reduzieren von Eisenoxidschichten einer Edelstahloberfläche.

In wenigstens einer Ausgestaltung umfasst das eingespritzte Reinigungsmittel alternativ oder zusätzlich wenigstens eine Komponente zur Ablösung von Eisenoxiden von einer Edelstahloberfläche. Spezielle Edelstahlreinigungsmittel zum Ablösen von Eisenoxiden ermöglichen es auch dickere oder ausgedehnte Beschichtungen von einer korrodierten Edelstahloberfläche zu entfernen.

In wenigstens einer Ausgestaltung werden die Schritte des Benetzens und Abführens unter aeroben Bedingungen in der Sterilisationskammer durchgeführt. In diesem Fall kann auf ein Absaugen von in der Sterilisationskammer befindlicher Luft verzichtet werden, was die Durchführung des Verfahrens beschleunigt.

In wenigstens einer alternativen Ausgestaltung werden die Schritte des Benetzens und Abführens unter anaeroben Bedingungen in der Sterilisationskammer durchgeführt, insbesondere durch Einleiten eines Schutzgases in die Sterilisationskammer. Eine derartige Reinigung kann insbesondere dann vorteilhaft sein, wenn zur Reinigung der Sterilisationskammer Chemikalien eingesetzt werden, die mit dem Luftsauerstoff reagieren. Solche ungewünschten Reaktionen können durch Einleitung eines Schutzgases wie beispielsweise Stickstoff, bevorzugt durch in einer Sterilisationsvorrichtung ohnehin vorhandene Komponenten, vermieden werden.

In wenigstens einer Ausgestaltung werden die Schritte des Benetzens und Abführens vollautomatisch und regelmäßig in Betriebspausen des Sterilisationsgerätes durchgeführt. Eine vollautomatische Reinigung in Betriebspausen, beispielsweise über Nacht oder am Wochenende, stellt sicher, dass das Sterilisationsgerät im normalen Betrieb, insbesondere am Tage, stets in einem hygienisch einwandfreien Zustand zur Verfügung steht. Wegen der vollständigen Automatisierung ist es dabei nicht erforderlich, dass ein geschulter Bediener während der Reinigung anwesend ist.

In wenigstens einer Ausgestaltung temperiert die Prozesssteuerung das eingespritzte Reinigungsmittel für einen vorbestimmten Temperaturbereich, insbesondere zwischen 75 und 95 °C, vorzugsweise zwischen 80 und 90 °C. Versuche mit verschiedenen Reinigungsmitteln haben ergeben, dass die genannten Temperaturbereiche für eine sanfte Reinigung der Sterilisationskammer besonders vorteilhaft sind. Zudem sind die genannten Temperaturbereiche durch typische Heizregelungen eines Sterilisationsgerätes leicht einzuhalten.

In wenigstens einer Ausgestaltung wiederholt die Prozesssteuerung die Schritte des Benetzens und Abführens für einen vorbestimmten Zeitraum, insbesondere zwischen 10 und 120 Minuten, bevorzugt zwischen 20 und 60 Minuten. Experimente mit unterschiedlichen Reinigungsprozessen haben ergeben, dass das wiederholte Einwirken eines geeigneten Reinigungsmittels für den oben genannten Zeitraum ausreichend ist, um bei regelmäßiger, beispielsweise wöchentlicher, Pflege den Aufbau von ungewünschten Oxidschichten zu vermeiden. Gleichzeitig sind die oben genannten Zeiträume kurz genug, um eine regelmäßige Reinigung der Sterilisationsvorrichtung in Arbeitspausen zu ermöglichen.

In wenigstens einer Ausgestaltung wählt die Prozesssteuerung eine Menge und/oder Konzentration des eingespritzten Reinigungsmittels in Abhängigkeit eines gewählten Reinigungsprogramms einer Mehrzahl von vorbestimmten Reinigungsprogrammen, insbesondere einem Grundreinigungsprogramm mit einer größeren Menge und/oder Konzentration des eingespritzten Reinigungsmittels und einem Pflegeprogramm mit einer geringeren Menge und/oder Konzentration des eingespritzten Reinigungsmittels. Durch eine derartige, programmabhängige Auswahl des eingespritzten Reinigungsmittels kann insbesondere zwischen einer regelmäßigen Wartungs- oder Pflegebetriebsart und einer gegebenenfalls nach größeren Verschmutzungen oder längeren Betriebsphasen erforderlichen Grundreinigung unterschieden werden.

In wenigstens einer Ausgestaltung führt die Prozesssteuerung zumindest zwei der folgenden Phasen eines Reinigungsprogramms aus:
- Dosierung des Reinigungsmittels;
- Beheizen der Sterilisationskammer und/oder des Reinigungsmittels;
- Reinigen der Sterilisationskammer durch das wiederholte Benetzen und Abführen des Reinigungsmittel;
- Spülen der Sterilisationskammer; und
- Repassivierung der Innenseite der Sterilisationskammer.

Der vollautomatische Ablauf mehrerer oder aller der oben genannten Phasen ermöglicht eine effektive Reinigung der Sterilisationskammer eines Sterilisationsgeräts.

Weitere vorteilhafte Ausgestaltungen der Erfindung sind in den angehängten Ansprüchen sowie der nachfolgenden Beschreibung von Ausführungsbeispielen offenbart.

Die Erfindung wird nachfolgend anhand von mehreren Ausführungsbeispielen unter Bezugnahme auf die angehängten Figuren im Detail beschrieben. Dabei werden jeweils unterschiedliche Aspekte der Erfindung unter Bezugnahme auf wenigstens ein konkretes Ausführungsbeispiel beschrieben. Dennoch lassen sich die jeweils im einzelnen beschriebenen Aspekte auch mit den anderen beschriebenen Ausführungsbeispielen kombinieren, soweit nichts Gegenteiliges angegeben ist. In den verschiedenen Ausführungsbeispielen werden dieselben Bezugszeichen für gleiche, gleichartige oder gleichwirkende Komponenten verwendet. Dies bedeutet jedoch nicht, dass die jeweils beschriebenen Komponenten verschiedener Ausführungsbeispielen völlig identisch sind.

In den Figuren zeigen:
- Figur 1: eine schematische Darstellung einer Sterilisationsvorrichtung gemäß einem ersten Ausführungsbeispiel,
- Figur 2: eine schematische Darstellung einer Sterilisationsvorrichtung gemäß einem zweiten Ausführungsbeispiel,
- Figur 3: eine schematische Darstellung einer Sterilisationsvorrichtung gemäß einem dritten Ausführungsbeispiel,
- Figur 4: schematisch einen Querschnitt durch eine Sterilisationskammer mit einer darin angeordneten Düse,
- Figur 5: schematisch eine perspektivische Ansicht einer Sterilisationskammer mit zwei unterschiedlichen Düsenpositionen,
- Figur 6A: eine fotografische Darstellung des Innenraums einer Sterilisationskammer mit einer fest eingebauten Düse,
- Figur 6B: eine Ausschnittvergrößerung der Darstellung aus Figur 6A,
- Figur 7: ein Ablaufdiagramm eines Reinigungsverfahrens zum Reinigen einer Sterilisationska mmer,
- Figur 8A: eine fotografische Darstellung einer Edelstahloberfläche mit darauf vorhandenen Eisenoxidschichten und
- Figur 8B: eine fotografische Darstellung der Edelstahloberfläche aus Figur 8A nach Durchführung einer vollautomatischen Reinigung gemäß einer Ausgestaltung der Erfindung.

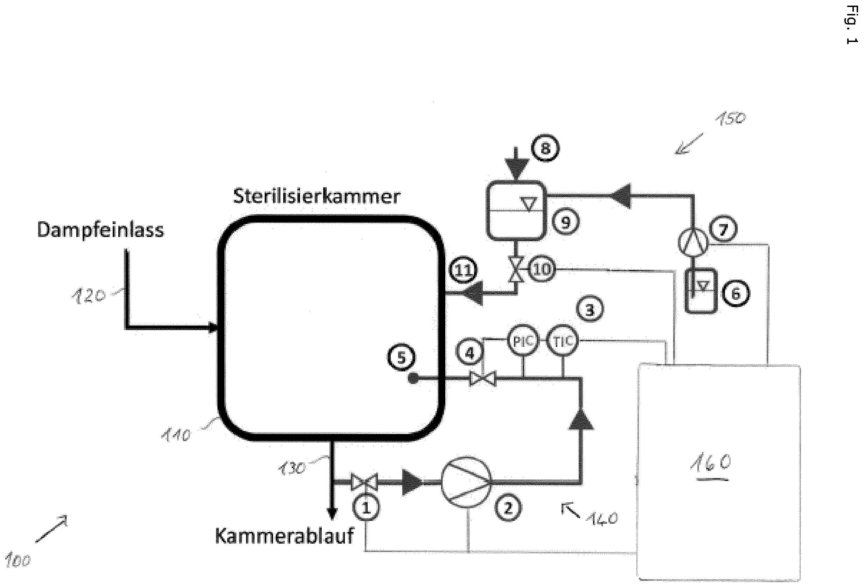

Figur 1 zeigt eine Sterilisationsvorrichtung 100 in Form eines Dampfsterilisators. Die Sterilisationsvorrichtung 100 umfasst unter anderem eine Sterilisationskammer 110, einen Dampfeinlass 120 und einen Kammerablauf 130. In einem Normalbetrieb, d. h. einer Betriebsart zum Sterilisieren von in der Sterilisationskammer 110 aufgenommenen Gut, wird der Innenraum der Sterilisationskammer 110 regelmäßig durch eine Vakuumpumpe evakuiert und nachfolgend durch den Einlass von Heißdampf aus vollentsalztem Wasser sterilisiert. Der genaue Ablauf derartiger Sterilisationsprozesse variiert in Abhängigkeit des zur sterilisierenden Guts sowie der baulichen Ausgestaltung der Sterilisationskammer 110. Auf eine weitere Darstellung von Details wird daher an dieser Stelle verzichtet.

Die in der Figur 1 dargestellte Sterilisationsvorrichtung 100 umfasst des Weiteren eine im Ausführungsbeispiel vollständig in die Sterilisationsvorrichtung integrierte Reinigungsvorrichtung, umfassend einen Umwälzkreislauf 140 sowie eine Dosiervorrichtung 150. Über die Dosiervorrichtung 150 wird, wie später im Detail beschrieben, ein flüssiges Reinigungsmittel in die Sterilisationskammer 110 eingeleitet. Über den Umwälzkreislauf 140 wird das in die Sterilisationskammer 110 eingeleitete Reinigungsmittel wiederholt in den Innenraum der Sterilisationskammer 110 eingespritzt um etwaige ungewünschte Oberflächenbeschichtungen, insbesondere Eisenoxidschichten, von einer Innenseite der Sterilisationskammer 110 zu entfernen.

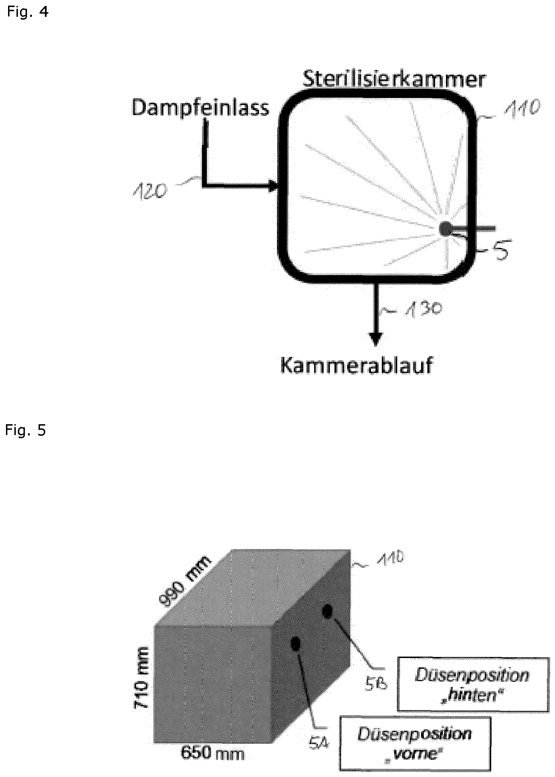

Hierzu ist der Umwälzkreislauf 140 mittels eines Absperrventils 1 mit dem Kammerablauf 130 verbunden. In einer Reinigungsbetriebsart öffnet das Absperrventil 1 eine fluidische Verbindung zwischen dem Kammerablauf 130 und einer Umwälzpumpe 2. Die Umwälzpumpe 2 befördert das Reinigungsmittel in Richtung einer in eine Wand der Sterilisationskammer 110 eingebrachten Sprühdüse 5. Optional kann die Umwälzpumpe 2 als Heizpumpe mit einem integrierten Heizelement zum direkten Beheizen des Reinigungsmittels ausgestaltet werden. Die Sprühdüse 5 wird zweckmäßigerweise so ausgelegt, dass sie fest in die Sterilisationsvorrichtung 100 eingebaut und während des normalen Sterilisationsbetriebs in der Sterilisationskammer 110 verbleiben kann. Bezüglich der genauen Anordnung und Ausgestaltung der Sprühdüse 5 wird auf die weiteren Ausführungen zu den Figuren 4-6B verwiesen.

Im Ausführungsbeispiel sind alle aktiven Komponenten der Sterilisationsvorrichtung 100 mit einer zentralen Prozesssteuerung 160, insbesondere einer speicherprogrammierbaren Industriesteuerung (SPS), verbunden. Dazu gehören auch Sensoren 3, umfassend einen Temperaturaufnehmer TIC und einen Druckaufnehmer PIC, die in einer Rohrleitung zwischen der Umwälzpumpe 2 und der Sprühdüse 5 angeordnet sind. Sensordaten der Sensoren 3 ermöglichen es der Prozesssteuerung 160, die Temperatur und den Druck des eingespritzt Reinigungsmittels zu erfassen und zu regeln. Hierzu ist im Ausführungsbeispiel ein Regelventil 4 der eigentlichen Sprühdüse 5 vorgelagert, über das die Prozesssteuerung 160 den Druck des eingespritzten Reinigungsmittels unabhängig von der maximalen Förderleistung der Umwälzpumpe 2 genau steuern kann. Alternativ oder zusätzlich kann die Prozesssteuerung 160 die Umwälzpumpe 2 direkt ansteuern, um den Druck des eingespritzten Reinigungsmittels zu regeln. Es ist auch möglich, weitere Sensordaten in die Prozesssteuerung mit einzubeziehen, z.B. eine Temperatur des Reinigungsmittels in der Kammer, einen Stand des Reinigungsmittels in der Kammer, die einen Rückschluss auf das Volumen des Reinigungsmittels zulässt, und/oder einen Druck in der Kammer.

Die Dosiervorrichtung 150 umfasst im dargestellten Ausführungsbeispiel einen Chemikalienvorratsbehälter 6, eine Chemikaliendosierungspumpe 7, einen Wasserzulauf 8, einen Vorlagebehälter 9, ein Zulaufventil 10 sowie einen Chemikalieneinlass 11.

In dem Chemikalienvorratsbehälter 6 ist eine hochkonzentrierte Reinigungslösung, insbesondere eine hochkonzentrierte Säure gespeichert. Im Betrieb wird von der Prozesssteuerung 160 zunächst ein Verdünnungsmittel, insbesondere vollentsalztes Wasser, über den Wasserzulauf 8 in den Vorlagebehälter 9 eingelassen. Dann wird von der Prozesssteuerung 160 mittels der Dosierpumpe 7 die hochkonzentrierte Reinigungslösung in den Vorlagebehälter 9 gepumpt und so auf eine gewünschte Konzentration verdünnt. Die beispielsweise auf eine Konzentration von 0,3-1% der hochkonzentrierten Reinigungslösung verdünnte Reinigungslösung stellt ein gebrauchsfertiges Reinigungsmittel dar. Nachfolgend wird eine gewünschte Menge des Reinigungsmittels über das Zulaufventil 10 und den Chemikalieneinlass 11 in die Sterilisierkammer eingeleitet, beispielsweise durch Wirkung der Schwerkraft. Die Menge des Reinigungsmittels wird durch die Öffnungsdauer des Zulaufventils 10 und/oder den Vorlagebehälter 9 und dessen Füllvolumen bestimmt und ist auf die Größe der Sterilisationsvorrichtung 100 abgestimmt. Bei einem typischen Dampfsterilisator für 6-9 Sterilisiereinheiten und einem Volumen von circa 450 Litern wird beispielsweise eine Menge von 8-16 Liter Reinigungsmittel angemischt.

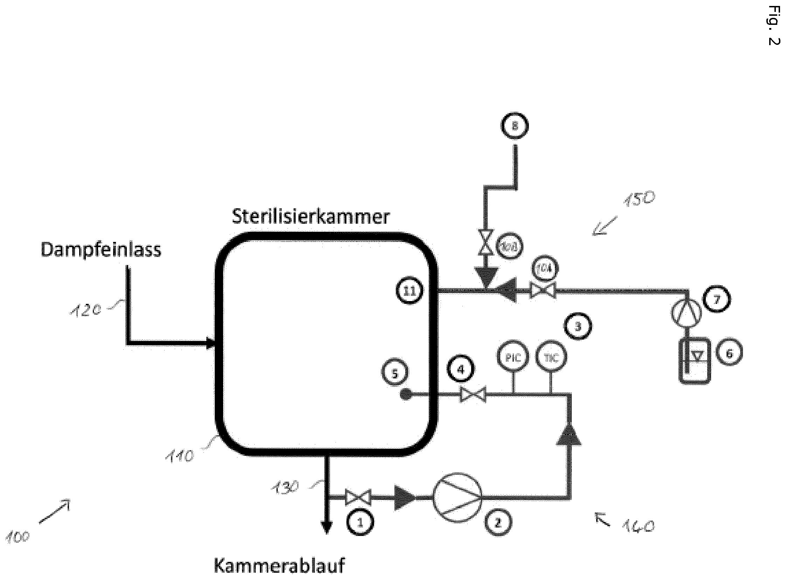

Figur 2 zeigt ein zweites Ausführungsbeispiel einer Sterilisationsvorrichtung 100. Auf eine wiederholte Beschreibung der bereits bezüglich der Figur 1 beschriebenen Merkmale wird aus Gründen einer kompakten Darstellung verzichtet. Zudem wurde die Prozesssteuerung 160 aus Gründen der Übersichtlichkeit weggelassen.

Die in der Figur 2 dargestellte Dosiervorrichtung 150 unterscheidet sich insbesondere dadurch von der Dosiervorrichtung 150 gemäß dem ersten Ausführungsbeispiel, dass sie keinen Vorlagebehälter, dafür aber zwei Zulaufventile 10A und 10B aufweist.

Beim Dosieren wird über die Dosierpumpe 7 und das erste Zulaufventil 10A die hochkonzentrierte Reinigungslösung in Richtung des Chemikalieneinlasses 11 gepumpt. Gleichzeitig wird über das zweite Zulaufventil 10B das Verdünnungsmittel, insbesondere vollentsalztes Wasser in denselben Abschnitt der Zuleitung eingeleitet, sodass das Reinigungsmittel am Chemikalieneinlass 11 in einer gewünschten Menge und mit einer gewünschten Konzentration austritt.

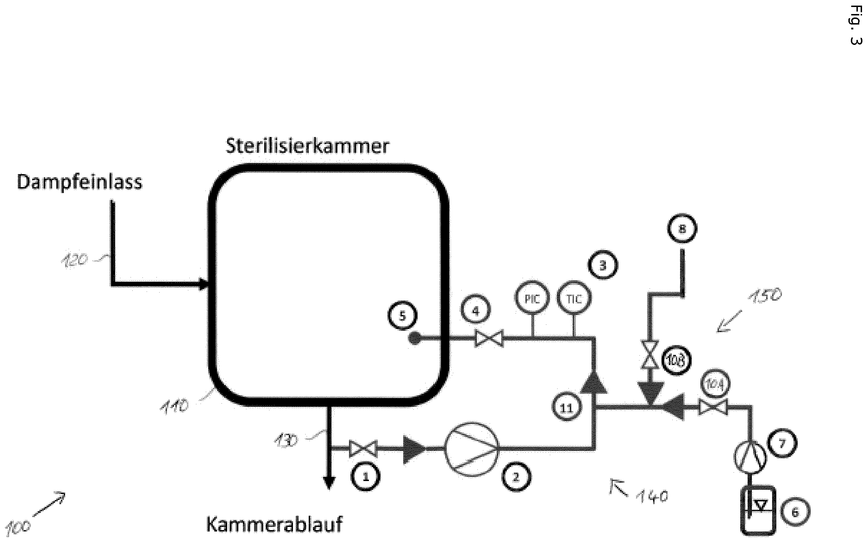

Figur 3 zeigt ein drittes Ausführungsbeispiel einer Sterilisationsvorrichtung 100. Auf eine wiederholte Beschreibung der bereits bezüglich der Figuren 1 und 2 beschriebenen Merkmale wird aus Gründen einer kompakten Darstellung erneut verzichtet.

Die in der Figur 3 dargestellte Sterilisationsvorrichtung 100 unterscheidet sich insbesondere dadurch von der Sterilisationsvorrichtung 100 gemäß dem zweiten Ausführungsbeispiel, dass das angemischte Reinigungsmittel direkt in den Umwälzkreislauf 140 eingeleitet wird. Auf diese Weise ist nur eine einzelne zusätzliche Öffnung in der Sterilisationskammer 110 für die Sprühdüse 5 erforderlich.

Figur 4 zeigt die Anordnung der Sprühdüse 5 in der Sterilisationskammer110. Die Sprühdüse 5 weist einen Sprühwinkel von beispielsweise 300° auf. Durch diese Maßnahmen ist sichergestellt, dass beim Einspritzen des Reinigungsmittels in die Sterilisationskammer 110 mit hinreichend hohen Druck fast die gesamte Innenoberfläche mit dem Reinigungsmittel benetzt wird. Lediglich im Bereich zwischen dem Kopf der Sprühdüse 5 und einem umgebenden Wandbereich ist ein direktes Aufbringen nicht möglich. Dennoch wirkt das Reinigungsmittel auch in diesem Bereich auf die Oberfläche der Sterilisationskammer 110 ein, indem Teile des von der Sprühdüse 5 nach oben abgegebenen Reinigungsmittels entlang der dargestellten Seitenwand der Sterilisationskammer 110 herablaufen und diese somit mit dem Reinigungsmittel benetzen.

Alternativ oder zusätzlich ist auch die Verwendung mehrerer Sprühdüsen möglich. Dies ist beispielhaft in der Figur 5 dargestellt, bei der zwei Sprühdüsen 5A und 5B an unterschiedlichen Positionen derselben Seitenwand eingebracht sind. Eine vordere Sprühdüse 5A sprühte große Bereiche einer quaderförmigen Sterilisationskammer 110 mit dem Reinigungsmittel ein. Zu dem von der vorderen Sprühdüse 5A eingesprühten Bereich zählt insbesondere auch ein Abschattungsbereich einer weiteren, hinteren Sprühdüse 5B. Umgekehrt sprüht die hintere Sprühdüse 5B einen Abschattungsbereich der vorderen Sprühdüse 5A ein.

Der Figur 5 sind des Weiteren typische Abmessungen der Sterilisationskammer 110 zu entnehmen. Beispielsweise handelt es sich um eine Kammer mit einer Breite von 650 mm, einer Höhe von 710 mm und einer Tiefe von 990 mm und somit einem Volumen von etwa einem halben Kubikmeter zur Aufnahme von Sterilisationsgut. In Sterilisationsvorrichtungen der angegebenen Größe wird das Sterilisationsgut typischerweise durch das Einfahren geeigneter Transportwagen in die Sterilisationskammer 110 eingebracht. Dabei ist es vorteilhaft, wenn sich die Sprühdüsen 5 in einem seitlichen Bereich der Sterilisationskammer 110 befinden.

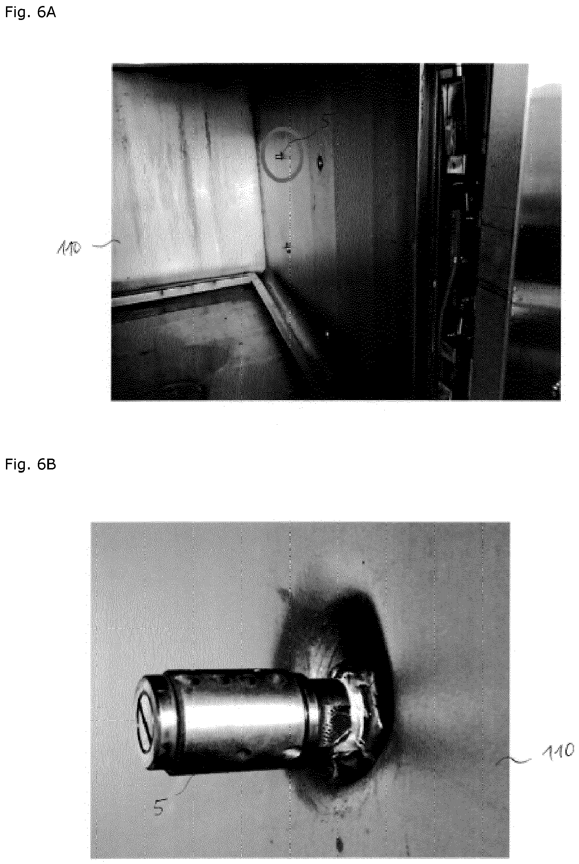

Dies ist in der fotografischen Darstellung gemäß Figur 6A ersichtlich. Dort ist im oberen, hinteren Teil einer Seitenwand einer Sterilisationskammer 110 eine Sprühdüse 5 fest angeordnet. Die Sprühdüse 5 ragt dabei nicht in einen gewöhnlichen Nutzraum der Sterilisationskammer 110 hinein, der im in der Figur 6A dargestellten Ausführungsbeispiel unten durch entsprechende Anschläge für den aufzunehmenden Transportwagen begrenzt wird.

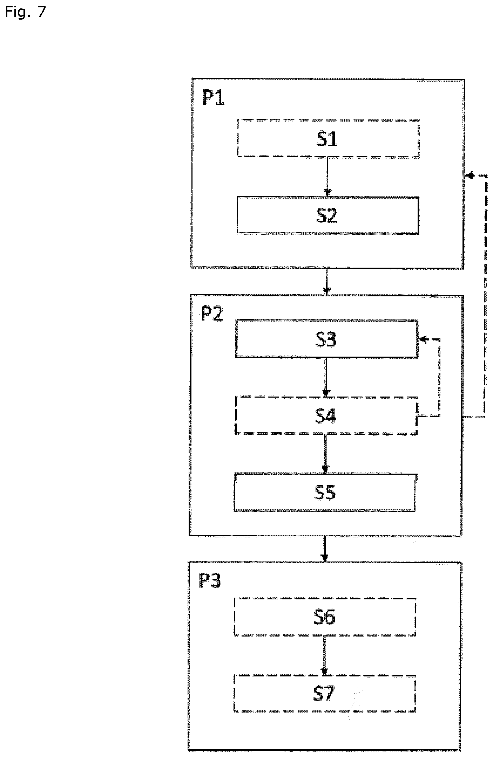

Figur 6B zeigt eine Ausschnittvergrößerung des in der Figur 6A umrandeten Bereiches. Darin ist zu erkennen, dass die Sprühdüse 5 durch eine vorhandene Kammerdurchführung in der Wand der Sterilisationskammer 110 geführt ist. Die Sprühdüse 5 umfasst einen rotierenden Kopf mit mehreren Sprühöffnungen. Durch eine entsprechend angewinkelte Öffnung wird der Kopf der Sprühdüse 5 durch Einleiten des Reinigungsmittels in eine Rotation versetzt, sodass eine gleichmäßige Verteilung des Reinigungsmittels in dem Innenraum der Sterilisationskammer 110 gewährleistet wird. Nachfolgend wird anhand der Figur 7 sowie der zuvor beschriebenen Ausführungsbeispiele der Ablauf eines Reinigungsprogramms für eine Sterilisationsvorrichtung 100 beschrieben. Das Reinigungsprogramm wird bevorzugt regelmäßig, beispielsweise zu einem vorbestimmten Zeitpunkt oder nach Ablauf einer vorbestimmten Anzahl von Sterilisationsprogrammen, gestartet. Hierzu wird die Sterilisationskammer 110 zunächst geleert.

In einer ersten Phase P1 wird das zur Reinigung verwendete Reinigungsmittel dosiert. Im Ausführungsbeispiel umfasst die Phase P1 einen optionalen Schritt S1 zum Anmischen des erforderlichen Reinigungsmittels und einen Schritt S2 zum Einleiteten des angemischten Reinigungsmittels in eine Sterilisationskammer 110. Diese Schritte wurden bereits bezüglich der Figuren 1 bis 3 beschrieben und werden daher hier nicht wiederholt. Bei chemischer Verträglichkeit können abweichend auch mehrere Reinigungsmittel gleichzeitig in die Sterilisationskammer 110 eingeleitet werden.

In einer zweiten Phase P2 wird eine innere Oberfläche mit dem angemischten Reinigungsmittel benetzt. Hierzu wird das Reinigungsmittel in einem Schritt S3 in die Sterilisationskammer 110 eingespritzt. In einem optionalen Schritt S4, der typischerweise parallel mit dem Schritt S3 ausgeführt wird, wird das aus der Sterilisationskammer 110 ablaufende Reinigungsmittel über einen Umwälzkreislauf 140 rezirkuliert. Anders als bei vielen konventioneller Reinigungsverfahren können die Schritte S3 und S4 in der Regel unter aeroben Verhältnissen, das heißt ohne vorherige Evakuierung der Sterilisationskammer 110 durchgeführt werden.

Typischerweise dauert die Phase P2 einen längeren Zeitraum, beispielsweise 60 Minuten, an. In dieser Zeit wird dasselbe Reinigungsmittel in den Schritten S3 und S4 wiederholt in die Sterilisationskammer 110 eingespritzt. Danach wird das in der Sterilisationskammer 110 befindliche Reinigungsmittel in einem Schritt S5 verworfen, zum Beispiel durch Verbinden des Kammerablaufs 130 mit einem Abflussrohr.

In den Schritten S3 und S4 kann die Temperatur bzw. der Druck des eingespritzt Reinigungsmittels optional von einer Steuervorrichtung geregelt werden. Beispielsweise kann die Innenseite der Sterilisationskammer 110 durch Einleiten von Dampf direkt in die Sterilisationskammer 110 oder in einen die Sterilisationskammer 110 umgebenen Doppelmantel auf eine gewünschte Temperatur zur Durchführung der Reinigung, beispielsweise 85 °C aufgeheizt werden. Auch ein Vorheizen der Sterilisationskammer 110 durch eine der eigentlichen Reinigung vorgelagerte Leersterilisation ist möglich. Alternativ oder zusätzlich ist auch eine direkte Beheizung des Reinigungsmittels über ein entsprechendes Heizelement möglich. Ein solches Vorheizen der Sterilisationskammer 110 oder Temperieren des Reinigungsmittels selbst haben den Vorteil, dass auch Teile der Sterilisationskammer, die nicht direkt beheizt werden können, wie beispielsweise eine Tür der Sterilisationskammer 110, auf eine für die Reinigung vorteilhafte Temperatur gebracht werden. Der Druck beim Einspritzen kann so gewählt werden, dass das Auftreffen des Reinigungsmittels auf einer Oberfläche der Sterilisationskammer 110 die chemische Reinigung durch den mechanischen Druck unterstützt. Dies ist jedoch in Abhängigkeit der übrigen Parameter nicht erforderlich.

Bei regelmäßiger Durchführung des beschriebenen Verfahrens reicht es in der Regel aus, die Phase P2 nur für einen einzelnen Reinigungszyklus unter Einsatz eines einzelnen Reinigungsmittels zu durchlaufen. Alternativ oder zusätzlich können, insbesondere bei hartnäckigen Oberflächenbeschichtungen, unterschiedliche Reinigungschemikalien sukzessive in die Sterilisationskammer 110 eingeleitet oder das eingesetzte Reinigungsmittel nach einer vorbestimmten Zeit gewechselt werden. In diesen Fällen wird das aktuell in der Sterilisationskammer 110 befindliche Reinigungsmittel im Schritt S5 verworfenen, in einer Wiederholung der Phase P1 ein neues Reinigungsmittel dosiert und in einem weiteren Durchlauf der Phase P2 verwendet. Die Phasen P1 und P2 und deren einzelne Verfahrensschritte können somit in Abhängigkeit der Anzahl der vorgesehenen Reinigungszyklen ein- oder mehrfach durchlaufen werden.

In einer abschließenden Phase P3 wird die Sterilisationskammer 110 für ihre normale Verwendung vorbereitet. Das sich zuvor in der Sterilisationskammer 110 befindliche Reinigungsmittel wurde bereits im Schritt S5 verworfen. Nachfolgend kann die Sterilisationskammer 110 in einem optionalen Schritt S6 mit einem Spülmittel, insbesondere vollentsalztem Wasser gespült werden. Dies kann beispielsweise durch Einleiten von Heißdampf über den Dampfeinlass 120 und dessen Kondensation an den Wänden der Sterilisationskammer 110 oder durch Einspritzen von vollentsalztem Wasser, beispielsweise über den Wasserzulauf 8, die Umwälzpumpe 2 und die Sprühdüse 5 erfolgen. Zusätzlich oder alternativ kann die gereinigte Oberfläche der Sterilisationskammer 110 in einem ebenfalls optionalen Schritt S7 repassiviert werden. Am Ende der Phase P3 steht die Sterilisationsvorrichtung erneut für einen Normalbetrieb zum Sterilisieren von in der Sterilisationskammer 110 eingebrachtem Gut zur Verfügung. Hierzu werden die für die Reinigung benötigten Komponenten so weit wie möglich von der Sterilisationskammer 110 getrennt, beispielsweise durch Schließen der Ventile 1 und 4 des Umwälzkreislaufes 140 und/oder der Zulaufventile 10 bzw. 10A und 10B der Dosiervorrichtung 150.

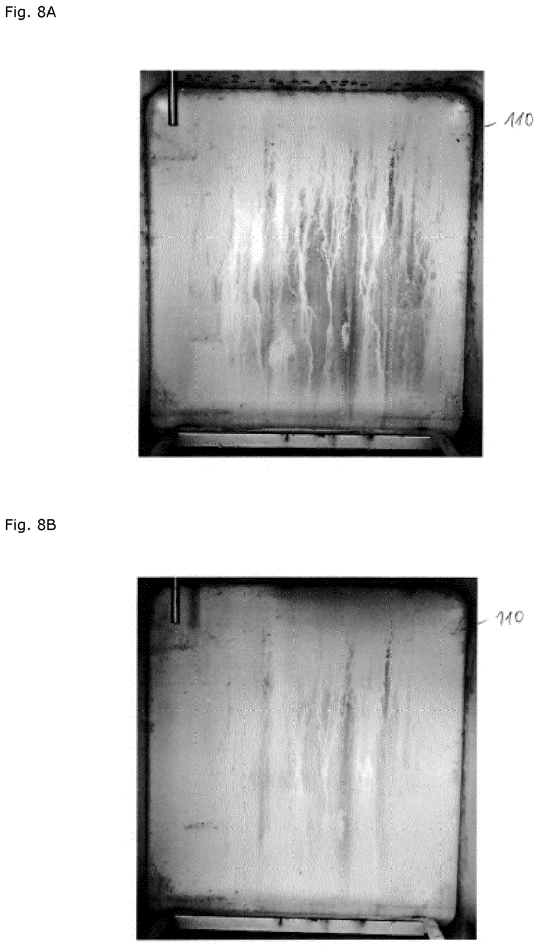

Die fotografischen Darstellungen in den Figuren 8A und 8B zeigen die Oberfläche des Innenraums einer Sterilisationskammer 110 vor beziehungsweise nach dem Durchlaufen des anhand von Figur 7 beschriebenen Reinigungsprogramms. Wie anhand der Figur 8B zu erkennen ist, stellt sich bereits nach einer knapp 30-minütigen Reinigung mit einem verhältnismäßig niedrig dosierten Reinigungsmittel eine deutliche Verbesserung der Oberflächeneigenschaften ein.

Wie eingangs ausgeführt weisen die zuvor beschriebenen Vorrichtung und Verfahren gegenüber bekannten Reinigungsvorrichtungen und -verfahren mehrere Vorteile auf. Insbesondere kann durch den regelmäßigen, vollautomatischen Einsatz einer in eine Sterilisationsvorrichtung integrierten Reinigungsvorrichtung eine regelmäßige, präventive Pflege aller relevanten Oberflächen sichergestellt werden. Da die Stärke der Ablagerungen durch die Wiederholung der Reinigung in kurzen Abständen minimiert wird, ist gegenüber bekannten Verfahren ein deutlich geringerer Chemikalien- und Zeiteinsatz nötig. Die Reinigung ist für die behandelten Oberflächen damit besonders schonend. Die Vorrichtung und das Verfahren ermöglichen zudem zügige Arbeitsabläufe und minimale Ausfallzeiten der Sterilisationsvorrichtung. Zudem kann der Einsatz von Chemikalien in Abhängigkeit der tatsächlichen Nutzung der Sterilisationsvorrichtung dosiert und damit im Regelfall reduziert werden. Eine regelmäßige, präventive Pflege von Edelstahloberflächen verlängert dabei zugleich die Lebensdauer der Sterilisationsvorrichtung.

### Bezugszeichenliste

- 1: Absperrventil
- 2: Umwälzpumpe
- 3: Sensoren
- 4: Regelventil
- 5, 5A, 5B: Sprühdüse
- 6: Chemikalienvorratsbehälter
- 7: Chemikaliendosierpumpe
- 8: Wasserzulauf
- 9: Vorlagebehälter
- 10, 10A, 10B: Zulaufventil
- 11: Chemikalieneinlauf
- 100: Sterilisationsvorrichtung
- 110: Sterilisationskammer
- 120: Dampfeinlass
- 130: Kammerablauf
- 140: Umwälzkreislauf
- 150: Dosiervorrichtung
- 160: Prozesssteuerung
- P1 bis P3: Phasen (des Reinigungsprogramms)
- S1 bis S7: Schritte (des Reinigungsprogramms)

## Patentansprüche

1. Sterilisationsvorrichtung (100), insbesondere Dampfsterilisator, umfassend:
- eine Sterilisationskammer (110) zur Aufnahme von zu sterilisierendem Gut in einer Normalbetriebsart;
- wenigstens eine Einspritzvorrichtung zum Einspritzen eines Reinigungsmittels in die Sterilisationskammer (110);
- wenigstens eine Ablaufvorrichtung zum Abführen des Reinigungsmittels aus der Sterilisationskammer (110); und
- eine Steuervorrichtung, die dazu eingerichtet ist, während einer Reinigungsbetriebsart Reinigungsmittel zum Reinigen der Sterilisationskammer (110) mittels der Einspritzvorrichtung in die Sterilisationskammer (110) einzuspritzen.

2. Sterilisationsvorrichtung (100) nach Anspruch 1, wobei die Einspritzvorrichtung wenigstens eine Pumpe (2) und wenigstens eine Düse (5) zum Einspritzen eines flüssigen Reinigungsmittels in die Sterilisationskammer (110) umfasst.

3. Sterilisationsvorrichtung (100) nach Anspruch 2, wobei die wenigstens eine Düse (5) innerhalb der Sterilisationskammer (110) in einem Bereich außerhalb eines Nutzraums der Sterilisationskammer (110) fest montiert ist, und/oder die wenigstens eine Düse (5) versenkbar in eine Wand der Sterilisationskammer (110) eingebaut ist.

4. Sterilisationsvorrichtung (100) nach einem der Ansprüche 2 oder 3, wobei die wenigstens eine Düse an einer Seitenwand der Sterilisationskammer (110) angeordnet ist.

5. Sterilisationsvorrichtung (100) nach einem der Ansprüche 2 bis 4, wobei die wenigstens eine Düse (5) beim Einspritzen des Reinigungsmittels rotiert und/oder wobei die wenigstens eine Düse auf einem rotierenden Sprüharm angeordnet ist.

6. Sterilisationsvorrichtung (100) nach einem der Ansprüche 2 bis 5, wobei die wenigstens eine Düse einen Sprühwinkel von mehr als 180°, bevorzugt mehr als 270° aufweist.

7. Sterilisationsvorrichtung (100) nach einem der Ansprüche 2 bis 5, wobei die Einspritzvorrichtung eine Mehrzahl von räumlich versetzt und/oder verdreht angeordneten Düsen (5A, 5B) umfasst.

8. Sterilisationsvorrichtung (100) nach einem der Ansprüche 2 bis 7, wobei die wenigstens eine Ablaufvorrichtung, die wenigstens eine Pumpe (2) und die wenigstens eine Düse (5) zusammen einen Umwälzkreislauf (140) zum wiederholten Benetzen einer Innenseite der Sterilisationskammer (110) mit dem flüssigen Reinigungsmittel bilden.

9. Sterilisationsvorrichtung (100) nach Anspruch 8, wobei die wenigstens eine Ablaufvorrichtung und die wenigstens eine Pumpe (2) über wenigstens ein erstes Ventil (1) miteinander verbunden sind, wobei das wenigstens eine erste Ventil (1) in der Reinigungsbetriebsart eine Rückführung des Reinigungsmittels über den Umwälzkreislauf (140) in die Sterilisationskammer (110) ermöglicht und in der Normalbetriebsart eine Rückführung von ablaufenden Flüssigkeiten in die Sterilisationskammer (110) unterbindet.

10. Sterilisationsvorrichtung (100) nach einem der Ansprüche 1 bis 9, weiter umfassend eine Dosiervorrichtung (150), wobei die Steuervorrichtung dazu eingerichtet ist, die Dosiervorrichtung (150) derart anzusteuern, dass in der Reinigungsbetriebsart eine vorbestimmte Menge des Reinigungsmittels in die Sterilisationskammer (110) eingeleitet wird.

11. Sterilisationsvorrichtung (100) nach Anspruch 10, wobei die Dosiervorrichtung (150) wenigstens einen Vorratsbehälter (6) zur Aufnahme wenigstens einer Chemikalie und wenigstens eine mit dem Vorratsbehälter (6) verbundene Dosiereinrichtung, insbesondere eine Dosierpumpe (7) und/oder ein zweites Ventil (10B), umfasst.

12. Sterilisationsvorrichtung (100) nach Anspruch 11, wobei der Vorratsbehälter (6) zur Aufnahme eines Reinigerkonzentrats und/oder eines ersten Reaktants zur Herstellung des Reinigungsmittels eingerichtet ist und die Dosiervorrichtung (150) des Weiteren eine Mischvorrichtung zum Mischen des Reinigerkonzentrats mit einem Verdünnungsmittel, insbesondere Wasser, bzw. des ersten Reaktants mit wenigstens einem zweiten Reaktant zur Herstellung des Reinigungsmittels in einem vorbestimmten Verhältnis umfasst.

13. Sterilisationsvorrichtung (200) nach einem der Ansprüche 10 bis 12, wobei die Dosiervorrichtung (150) direkt mit der Einspritzvorrichtung verbunden ist und die vorbestimmte Menge des Reinigungsmittels von der Dosiervorrichtung (150) über die Einspritzvorrichtung in die Sterilisationskammer (110) eingebracht wird.

14. Sterilisationsvorrichtung (100) nach einem der Ansprüche 10 bis 12, wobei die Dosiervorrichtung (150) über einen von der Einspritzvorrichtung gesonderten Zulauf (11) mit der Sterilisationskammer (110) verbunden ist und die vorbestimmte Menge des Reinigungsmittels von der Dosiervorrichtung (150) über den Zulauf (11) in die Sterilisationskammer (110) eingebracht wird.

15. Sterilisationsvorrichtung (100) nach einem der Ansprüche 1 bis 14, weiter umfassend wenigstens einen Sensor (3), wobei die Steuervorrichtung des Weiteren dazu eingerichtet ist, den Druck bzw. die Temperatur des Reinigungsmittels in Abhängigkeit von erfassten Sensordaten während der Reinigungsbetriebsart zu regeln.

16. Sterilisationsvorrichtung (100) nach Anspruch 15, wobei die Sterilisationskammer (110) beheizbar ist, insbesondere durch Einleiten von Heißdampf in einen Doppelmantel der Sterilisationskammer (110) und/oder durch direktes Einleiten von Heißdampf in die Sterilisationskammer (110), und die Steuervorrichtung dazu eingerichtet ist, die Temperatur des Reinigungsmittels zumindest teilweise über eine Beheizung der Sterilisationskammer (110) zu regeln.

17. Sterilisationsvorrichtung (100) nach Anspruch 15 oder 16, wobei die Einspritzvorrichtung ein von der Sterilisationskammer (110) gesondertes Heizelement zum Beheizen des Reinigungsmittels vor dem Einspritzen in die Sterilisationskammer (110) umfasst und die Steuervorrichtung dazu eingerichtet ist, die Temperatur des Reinigungsmittels zumindest teilweise über eine Beheizung des Reinigungsmittels durch das Heizelement zu regeln.

18. Sterilisationsvorrichtung (100) nach einem der Ansprüche 15 bis 17, wobei die Einspritzvorrichtung des Weiteren ein Regelventil (4) zur Druckregulierung umfasst, und die Steuervorrichtung dazu eingerichtet ist, den Druck des eingespritzten Reinigungsmittels über das Regelventil (4) zu regeln.

19. Sterilisationsvorrichtung (100) nach einem der Ansprüche 1 bis 18, weiter umfassend wenigstens eine Spülvorrichtung zum Spülen der Sterilisationskammer (110) mit einem chemisch neutralen Spülmittel, insbesondere Wasser, wobei die Steuervorrichtung des Weiteren dazu eingerichtet ist, die Sterilisationskammer (110) nach dem Einspritzen des Reinigungsmittels mit dem Spülmittel zu spülen.

20. Sterilisationsvorrichtung (100) nach einem der Ansprüche 1 bis 19, wobei die Steuervorrichtung als programmierbare Prozesssteuerung (160) ausgestaltet ist, die zur Durchführung wenigstens eines Reinigungsprogramms und wenigstens eines Sterilisationsprogramms programmiert ist.

21. Sterilisationsvorrichtung (100) Anspruch 20, wobei die Prozesssteuerung (160) dazu eingerichtet ist, das wenigstens eine Reinigungsprogramm regelmäßig, insbesondere nach einer vorbestimmten Anzahl von durchgeführten Sterilisationsprogrammen, nach Ablauf einer vorbestimmten Zeitspanne und/oder zu einem vorbestimmten, wiederkehrenden Zeitpunkt durchzuführen.

22. Verfahren zum Reinigen einer Sterilisationskammer (110) eines Sterilisationsgeräts, insbesondere zum Entfernen von Eisenoxiden von einer Edelstahloberfläche der Sterilisationskammer (110), mit den Schritten:
- Benetzen einer Innenseite der Sterilisationskammer (110) mit einem über eine in die Sterilisationskammer (110) integrierte Einspritzvorrichtung eingespritzten Reinigungsmittel; und
- Abführen des in die Sterilisationskammer (110) eingespritzten Reinigungsmittels über wenigstens eine Ablaufvorrichtung der Sterilisationskammer (110), wobei die Schritte des Benetzens und Abführens durch eine Prozesssteuerung (160) des Sterilisationsgeräts wiederholt durchgeführt werden.

23. Verfahren nach Anspruch 22, wobei das eingespritzte Reinigungsmittel eine Säure, insbesondere eine wässrige Lösung, umfasst, insbesondere mit einem pH-Wert von weniger als 5, bevorzugt mit einem pH-Wert in einem Bereich zwischen 2 und 4.

24. Verfahren nach Anspruch 22 oder 23, wobei das eingespritzte Reinigungsmittel wenigstens eine Komponente zur Ablösung von Eisenoxiden von einer Edelstahloberfläche umfasst.

25. Verfahren nach einem der Ansprüche 22 bis 24, wobei die Schritte des Benetzens und Abführens unter aeroben Bedingungen in der Sterilisationskammer (110) durchgeführt werden.

26. Verfahren nach einem der Ansprüche 22 bis 24, wobei die Schritte des Benetzens und Abführens unter anaeroben Bedingungen in der Sterilisationskammer (110), insbesondere durch Einleiten eines Schutzgases in die Sterilisationskammer (110), durchgeführt werden.

27. Verfahren nach einem der Ansprüche 22 bis 26, wobei die Schritte des Benetzens und Abführens vollautomatisch und regelmäßig in Betriebspausen des Sterilisationsgeräts durchgeführt werden.

28. Verfahren nach einem der Ansprüche 22 bis 27, wobei die Prozessteuerung (160) das eingespritzte Reinigungsmittel für einen vorbestimmten Temperaturbereich, insbesondere zwischen 75 °C und 95 °C, vorzugsweise zwischen 80 °C und 90 °C, temperiert.

29. Verfahren nach einem der Ansprüche 22 bis 28, wobei die Prozessteuerung (160) die Schritte des Benetzens und Abführens für einen vorbestimmten Zeitraum, insbesondere zwischen 10 und 120 Minuten, bevorzugt zwischen 20 und 60 Minuten, wiederholt.

30. Verfahren nach einem der Ansprüche 22 bis 29, wobei die Prozessteuerung (160) eine Menge und/oder Konzentration des eingespritzten Reinigungsmittels in Abhängigkeit eines gewählten Reinigungsprogramms einer Mehrzahl von vorbestimmten Reinigungsprogrammen wählt, insbesondere einem Grundreinigungsprogramm mit einer größeren Menge und/oder Konzentration des eingespritzten Reinigungsmittels und einem Pflegeprogramm mit einer geringeren Menge und/oder Konzentration des eingespritzten Reinigungsmittels.

31. Verfahren nach einem der Ansprüche 22 bis 30, wobei die Prozessteuerung (160) zumindest zwei der folgenden Phasen eines Reinigungsprogramms ausführt:
- Dosieren des Reinigungsmittels;
- Beheizen der Sterilisationskammer (110) und/oder des Reinigungsmittel;
- Reinigen der Sterilisationskammer (110) durch das wiederholte Benetzen und Abführen des Reinigungsmittels;
- Spülen der Sterilisationskammer (110); und
- Repassivierung der Innenseite der Sterilisationskammer (110).
